# EUROPÄISCHE PATENTSCHRIFT

(11) **EP 1 531 898 B1**
(45) Veröffentlichungstag und Bekanntmachung des Hinweises auf die Patenterteilung: **22.11.2006**
(21) Anmeldenummer: 03775131.0
(22) Anmeldetag: 20.08.2003
(51) Int. Cl.: A61N 1/30

(54) **MESSELEKTRODENANORDNUNG**
MEASURING ELECTRODE SYSTEM
SYSTEME D'ELECTRODE DE MESURE

(30) Priorität: 21.08.2002 DE 10238310; 26.11.2002 WO PCT/EP02/13327; 15.01.2003 DE 10301258
(43) Veröffentlichungstag der Anmeldung: 25.05.2005
(73) Patentinhaber: Viasys Healthcare GmbH, 97204 Höchberg (DE)
(72) Erfinder: EICHLER, Rüdiger, 97225 Zellingen (DE)
(74) Vertreter: Beier, Ralph
(86) Internationale Anmeldenummer: PCT/EP2003/009228
(87) Internationale Veröffentlichungsnummer: WO 2004/017829

(56) Entgegenhaltungen:
- EP-A- 0 571 712
- US-A- 4 842 577
- US-A- 5 362 308
- US-A- 5 496 266

## Beschreibung

Die Erfindung betrifft eine Messelektrodenanordnung, insbesondere für die Elektro-Impedanz-Tomographie, gemäß dem Oberbegriff des Anspruchs 1.

Zur Durchführung der sogenannten Elektro-Impedanz-Tomographie (EIT) bei einem Patienten müssen zahlreiche Messelektroden an dem zu untersuchenden Körperteil des Patienten befestigt werden, wie beispielsweise am Brustkorb des Patienten. Die Messelektroden müssen hierbei möglichst genau positioniert werden und ihre Position während des Messvorgangs möglichst exakt beibehalten werden, da Fehlpositionierungen der Messelektroden das Messergebnis verfälschen würden. Darüber hinaus müssen die Messelektroden den zu untersuchenden Körperteil elektrisch möglichst gut kontaktieren, d.h. der Übergangswiderstand zwischen den Messelektroden und den zu untersuchenden Körperteilen soll möglichst gering sein und darf während des Messvorgangs nicht schwanken.

Es sind deshalb Messelektrodenanordnungen für die Elektro-Impedanz-Tomographie bekannt, bei denen mehrere Elektroden an der Körperoberfläche des Patienten fest geklebt werden, wodurch eine Veränderung der Elektrodenposition während des Messvorgangs verhindert wird. Die elektrische Kontaktierung des zu untersuchenden Körperteils des Patienten kann sich hierbei jedoch während des Messvorgangs oder zwischen mehreren aufeinanderfolgenden Messvorgängen ändern, wenn eine zwischen der aufgeklebten Elektrode und der Körperoberfläche befindliche Kontaktflüssigkeit austrocknet.

Nachteilig an den bekannten Messelektrodenanordnungen für die Elektro-Impedanz-Tomographie ist deshalb die unbefriedigende elektrische Kontaktierung des Messobjekts.

Aus EP-A-0 571 712 ist eine Messelektrodenanordnung für die Elektroimpedanztomographie bekannt, die mindestens eine Elektrode zur elektrischen Kontaktierung eines Objekts aufweist, wobei auf der dem Objekt abgewandten Seite der Elektrode ein Vorratsraum angeordnet ist, der ein Kontaktmittel zur Verringerung des elektrischen Übergangswiderstands zwischen der Elektrode und dem Objekt enthält, wobei die Elektrode für das Kontaktmittel durchlässig ist. Diese bekannte Messelektrodenanordnung ist jedoch für die Iontophorese geeignet, wohingegen die Erfindung auf eine Messelektrodenanordnung für die Elektro-Impedanz-Tomographie abzielt.

Ferner sind aus US-A-4 842 577, US-A-5 496 266 und US-A-5 362 308 Elektrodenanordnungen für die Iontophorese bekannt.

Der Erfindung liegt deshalb die Aufgabe zu Grunde, eine Messelektrodenanordnung zu schaffen, die sich insbesondere für die Elektro-Impedanz-Tomographie eignet und eine möglichst gute elektrische Kontaktierung des Messobjektes ermöglicht.

Die Erfindung wird, ausgehend von der eingangs beschriebenen bekannten Messelektrodenanordnung gemäß dem Oberbegriff des Anspruchs 1, durch die kennzeichnenden Merkmale des Anspruchs 1 gelöst.

Die Erfindung umfasst die allgemeine technische Lehre, einen Vorratsraum für ein Kontaktmittel in die Messelektrodenanordnung zu integrieren, wobei das Kontaktmittel den elektrischen Übergangswiderstand zwischen der Messelektrode und dem Messobjekt verringert.

Erfindungsgemäß ist der Vorratsraum für das Kontaktmittel auf der dem Messobjekt abgewandten Seite der Messelektrode angeordnet, wobei die Messelektrode für das Kontaktmittel mindestens teilweise durchlässig ist, so dass das Kontaktmittel aus dem Vorratsraum in den Zwischenraum zwischen der Oberfläche des Messobjekts und der Messelektrode eindringen kann.

Bei dem Kontaktmittel zur Verringerung des elektrischen Übergangswiderstands kann es sich beispielsweise um eine Flüssigkeit, ein Gel, einen Schaum oder eine Paste handeln. Die Erfindung ist jedoch nicht auf diese Arten von Kontaktmitteln beschränkt, sondern auch mit anderen Stoffen realisierbar, die zu einer Verringerung des elektrischen Übergangswiderstands zwischen der Messelektrode und dem Messobjekt beitragen.

Hierbei ist die Messelektrode nur für einzelne Bestandteile des Kontaktmittels durchlässig, wohingegen die Messelektrode für die restlichen Bestandteile des Kontaktmittels undurchlässig ist. So enthält das Kontaktmittel Ionen in wässriger Lösung, wobei die Ionen durch die Messelektrode hindurch diffundieren können, wohingegen die als Lösungsmittel verwendete Flüssigkeit in dem Vorratsraum zurückgehalten wird.

Die Befestigung der erfindungsgemäßen Messelektrodenanordnung an dem Messobjekt erfolgt vorzugsweise durch eine Verklebung. Hierzu ist vorzugsweise auf der dem Messobjekt zugewandten Seite der Messelektrodenanordnung eine Klebeschicht angeordnet, um die Messelektrodenanordnung an dem Messobjekt zu fixieren. Die Erfindung ist jedoch nicht auf diese Art der Befestigung der Messelektrodenanordnung an dem Messobjekt beschränkt. Es bestehen vielmehr auch andere Möglichkeiten der mechanischen Fixierung der Messelektrodenanordnung an dem Messobjekt, beispielsweise mittels eines gürtelförmigen Elektrodenträgers, der um den Brustkorb des Patienten herum gelegt wird und die einzelnen Messelektroden in einer vorgegebenen geometrischen Konfiguration fixiert. Der Vorratsraum für das Kontaktmittel wird vorzugsweise durch eine Kunststoffschicht begrenzt, die vorzugsweise auf der dem Messobjekt abgewandten Seite der Messelektrode angeordnet ist. Eine derartige Kunststoffschicht kann beispielsweise aus Polyethylen (PE) bestehen, jedoch sind auch andere Materialien möglich. Die Befestigung der als Begrenzung des Vorratsraums dienenden Kunststoffschicht an der Messelektrode erfolgt vorzugsweise durch Hitzeverschweißen, oder Ultraschallverschweißen, jedoch sind auch andere Herstellungsverfahren denkbar.

Darüber hinaus weist die erfindungsgemäße Messelektrodenanordnung mindestens eine elektrische Abschirmung auf, die aus einem elektrisch leitfähigen Material besteht und gegenüber der Messelektrode elektrisch isoliert ist. Eine derartige elektrische Abschirmung dient beim Einsatz der erfindungsgemäßen Messelektrodenanordnung als Stimulationselektrode im Rahmen der Elektro-Impedanz-Tomographie dazu, die in der Umgebung befindlichen Messelektroden vor dem durch die Stimulation verursachten Störfeld abzuschirmen. Bei einem Einsatz der erfindungsgemäßen Messelektrodenanordnung zur Erfassung der im Rahmen der Elektro-Impedanz-Tomographie entstehenden Potentialteilungen dient die Abschirmung hingegen dazu, die durch die möglicherweise benachbarten Stimulationselektroden hervorgerufenen Störfelder abzuschirmen.

In einer Ausführungsform weist die erfindungsgemäße Messelektrodenanordnung mehrere Messelektroden auf, die zueinander elektrisch isoliert sind und dadurch eine getrennte Messung beziehungsweise Stimulation ermöglichen.

Hierbei sind vorzugsweise auch mehrere elektrische Abschirmungen vorgesehen, die zueinander und zu den einzelnen Messelektroden elektrisch isoliert sind. Eine derartige Anordnung mit mehreren getrennten elektrischen Abschirmungen bietet die Möglichkeit, die Abschirmungen gezielt mit einem elektrischen Signal zu beaufschlagen, um die Abschirmwirkung zu verbessern. Bei einer Verwendung einer Messelektrode als Stimulationselektrode kann die zugehörige Abschirmung beispielsweise gezielt mit einem elektrischen Signal beaufschlagt werden, welches das durch die Stimulation hervorgerufene Störfeld kompensiert.

Es ist jedoch alternativ auch möglich, dass die erfindungsgemäße Messelektrodenanordnung eine gemeinsame elektrische Abschirmung für sämtliche Messelektroden aufweist, wobei die gemeinsame Abschirmung vorzugsweise auf Massepotential gelegt wird.

In der erfindungsgemäßen Messelektrodenanordnung ist die Abschirmung vorzugsweise auf der dem Messobjekt abgewandten Seite der Messelektrode angeordnet, um eine möglichst gute Abschirmwirkung zu erreichen.

In einem bevorzugten Ausführungsbeispiel sind die einzelnen Messelektroden an einen gürtelförmigen Elektrodenträger angeordnet, wobei der Elektrodenträger zur Einstellung des Elektrodenabstands dehnbar ist.

Schließlich umfasst die Erfindung auch die Verwendung der erfindungsgemäßen Messelektrodenanordnung bei der Elektro-Impedanz-Tomographie.

Bei dem zu untersuchenden Messobjekt handelt es sich hier vorzugsweise um einen Brustkorb eines Patienten, wobei die erfindungsgemäße Messelektrodenanordnung an dem Brustkorb befestigt wird, um die Elektro-Impedanz-Tomographie durchführen zu können. Die Erfindung ist jedoch hinsichtlich des zu untersuchenden Messobjektes nicht auf einen Brustkorb eines Patienten beschränkt, sondern grundsätzlich auch bei anderen Körperteilen einsetzbar.

Andere vorteilhafte Weiterbildungen der Erfindungen sind in Unteransprüchen gekennzeichnet und werden nachfolgend in der Beschreibung eines bevorzugten Ausführungsbeispiels der Erfindung näher erläutert.

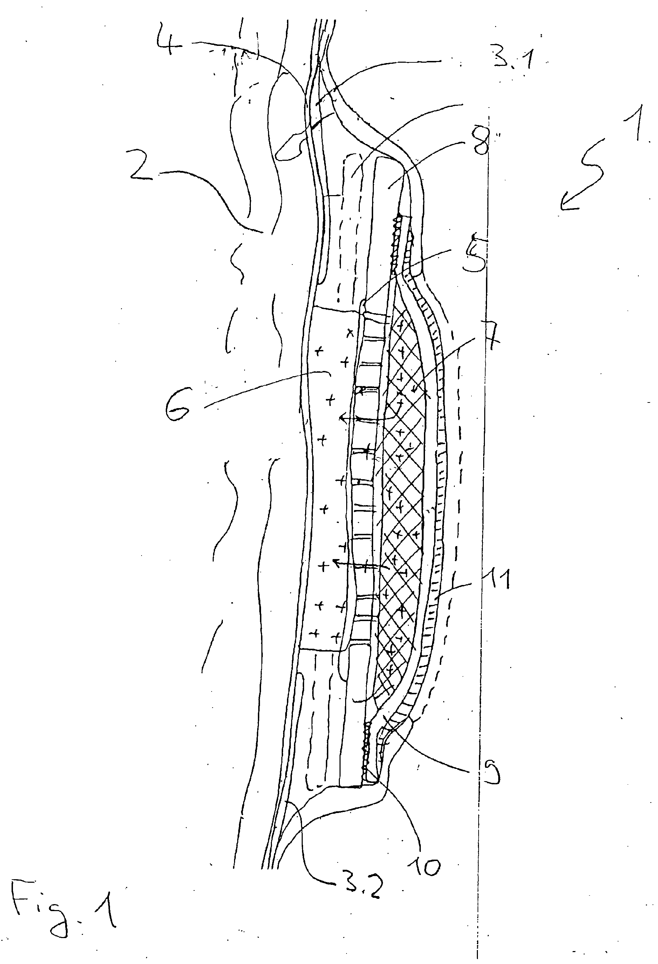

Figur 1 eine Querschnittsansicht einer erfindungsgemäßen Elektrodenanordnung.

Die in Figur 1 dargestellte Messelektrodenanordnung 1 dient zur elektrischen Kontaktierung eines Brustkorbs 2 eines Patienten bei der Elektro-Impedanz-Tomographie.

Die Elektrodenanordnung 1 ist derzeit gürtelförmig ausgebildet wird um den Brustkorb 2 des Patienten herum gelegt und mittels zweier Klebestreifen 3.1, 3.2 auf der Hautoberfläche 4 des Patienten festgeklebt. Durch diese mechanische Fixierung der Elektrodenanordnung 1 auf der Körperoberfläche 4 wird verhindert, dass sich die Positionierung der Messelektrodenanordnung 1 während der Elektro-Impedanz-Tomographie oder zwischen mehreren aufeinanderfolgenden Tomographien ändert, wodurch das Messergebnis verfälscht wird.

In der Messelektrodenanordnung 1 befinden sich über dem Umfang des Brustkorbs 2 verteilt mehrere Messelektroden, wobei in der Querschnittsansicht nur eine Messelektrode 5 dargestellt ist. Die Messelektrode 5 liegt hierbei nicht direkt auf der Körperoberfläche 4 auf, sondern ist gegenüber der Körperoberfläche 4 beabstandet angeordnet. Bei der Elektro-Impedanz-Tomographie ist der Zwischenraum zwischen der Messelektrode 5 und der Körperoberfläche 4 durch ein elektrisch leitfähiges Gel 6 aufgefüllt, das Ionen enthält und dadurch eine gute elektrische Kontaktierung der Körperoberfläche 4 bewirkt.

Bei einem lang andauernden Messvorgang oder bei langen Pausen zwischen aufeinanderfolgenden Messvorgängen besteht die Gefahr, dass das Gel 6 in dem Zwischenraum zwischen Elektrode 5 und der Körperoberfläche 4 austrocknet, worunter die elektrische Kontaktierung leiden würde. Die erfindungsgemäße Messanordnung 1 weist deshalb auf der dem Brustkorb abgewandten Seite der Messelektrode 5 einen Vorratsraum 7 auf, der ein elektrisch leitfähiges Gel enthält. Die in dem Vorratsraum 7 in dem Gel 6 befindlichen Ionen können hierbei durch die Messelektrode 5 hindurch in den Zwischenraum zwischen der Messelektrode 5 und der Körperoberfläche 4 diffundieren, um die Leitfähigkeit des Gels 6 und damit die elektrische Kontaktierung der Körperoberfläche 4 auf einem möglichst konstanten Niveau zu halten. Die Messelektrode 5 ist deshalb für die in dem Gel 6 befindlichen Ionen durchlässig, wohingegen die Messelektrode 5 für das Gel 6 ansonsten undurchlässig ist.

Weiterhin weist die erfindungsgemäße Messelektrodenanordnung 1 einen Elektrodenträger 8 auf, an dem auf der dem Brustkorb 2 abgewandten Seite eine Kunststoffschicht 9 befestigt ist, wobei die Kunststoffschicht 9 den Vorratsraum 7 begrenzt. Die Befestigung der Kunststoffschicht 9 an dem Elektrodenträger 8 erfolgt hierbei durch eine Hitzeverschweißung 10.

Schließlich weist die erfindungsgemäße Messelektrodenanordnung 1 noch eine elektrische Abschirmung 11 auf, die auf der dem Vorratsraum 7 abgewandten Seite der Kunststoffschicht 9 angebracht ist und aus einem elektrisch leitfähigen Material besteht. Hierbei ist für jede der über den Umfang des Brustkorbs 2 verteilt angeordneten Messelektroden 5 jeweils eine Abschirmung 11 vorgesehen, wobei die einzelnen Abschirmungen 11 zueinander und zu der Messelektrode 5 elektrisch isoliert sind. Die einzelnen Abschirmungen 11 können deshalb zur Abschirmung von Störfeldern gezielt mit einem elektrischen Signal beaufschlagt werden. Es ist jedoch auch möglich, die einzelnen Abschirmungen 11 einfach an Masse zu legen.

Die Erfindung ist nicht auf das vorstehend beschriebene bevorzugte Ausführungsbeispiel beschränkt. Vielmehr ist eine Vielzahl von Varianten und Abwandlungen möglich, die ebenfalls von dem Erfindungsgedanken Gebrauch machen und deshalb in den Schutzbereich fallen.

## Patentansprüche

1. Messelektrodenanordnung (1) für die Elektro-Impedanz-Tomographie, mit mindestens einer Messelektrode (5) zur elektrischen Kontaktierung eines Messobjekts (2), wobei auf der dem Messobjekt (2) abgewandten Seite der Messelektrode (5) ein Vorratsraum (7) angeordnet ist, der ein Kontaktmittel (6) zur Verringerung des elektrischen Übergangswiderstands zwischen der Messelektrode (5) und dem Messobjekt (2) enthält, wobei die Messelektrode (5) für das Kontaktmittel (6) mindestens teilweise durchlässig ist, während das Kontaktmittel (6) Ionen in wässriger Lösung enthält,
**dadurch gekennzeichnet, dass**
die Ionen durch die Messelektrode (5) hindurch diffundieren können, wohingegen die Messelektrode (5) für die als Lösungsmittel verwendete Flüssigkeit undurchlässig ist.

2. Messelektrodenanordnung (1) nach Anspruch 1, **dadurch gekennzeichnet, dass** das Kontaktmittel (6) eine Flüssigkeit, ein Gel, ein Schaum oder eine Paste ist.

3. Messelektrodenanordnung (1) nach mindestens einem der vorhergehenden Ansprüche, **dadurch gekennzeichnet, dass** auf der dem Messobjekt (2) zugewandten Seite der Messelektrodenanordnung (1) eine Klebeschicht (3.1, 3.2) angeordnet ist, um die Messelektrodenanordnung (1) an dem Messobjekt (2) zu fixieren.

4. Messelektrodenanordnung (1) nach mindestens einem der vorhergehenden Ansprüche, **dadurch gekennzeichnet, dass** der Vorratsraum (7) durch eine Kunststoffschicht (9) begrenzt ist.

5. Messelektrodenanordnung (1) nach mindestens einem der vorhergehenden Ansprüche, **gekennzeichnet durch** mindestens eine elektrischen Abschirmung (11), die aus einem elektrisch leitfähigen Material besteht und gegenüber der Messelektrode (5) elektrisch isoliert ist.

6. Messelektrodenanordnung (1) nach Anspruch 5, **gekennzeichnet durch** mehrere zueinander elektrisch isolierte Messelektroden (5).

7. Messelektrodenanordnung (1) nach Anspruch 6, **gekennzeichnet durch** mehrere zueinander elektrisch isolierte Abschirmungen (11), wobei die Abschirmungen (11) jeweils an einer der Messelektroden (5) angeordnet sind.

8. Messelektrodenanordnung (1) nach Anspruch 6, **gekennzeichnet durch** eine gemeinsame elektrische Abschirmung (11) für die Messelektroden (5).

9. Messelektrodenanordnung (1) nach mindestens einem der Ansprüche 5 bis 8, **dadurch gekennzeichnet, dass** die Abschirmung (11) auf der dem Messobjekt (2) abgewandten Seite der Messelektrode (5) angeordnet ist.

10. Messelektrodenanordnung (1) nach mindestens einem der Ansprüche 5 bis 9, **dadurch gekennzeichnet, dass** die Messelektroden (5) an einem gürtelförmigen Elektrodenträger (8) angebracht sind, wobei der Elektrodenträger (8) zur Einstellung des Elektrodenabstands dehnbar ist.

11. Verwendung einer Messelektrodenanordnung (1) nach mindestens einem der vorhergehenden Ansprüche für die Elektro-Impedanz-Tomographie.

## Claims

1. Measuring electrode arrangement (1) for electric impedance tomography, with at least one measuring electrode (5) for electric contacting of a measurement object (2), wherein on the side of the measuring electrode (5) facing away from the measurement object (2) there is disposed a storage chamber (7) which contains a contact means (6) for the reduction of the electrical transition impedance between the measuring electrode (5) and the measurement object (2), wherein the measuring electrode (5) is at least partially permeable to the contact means (6), whilst the contact means (6) contains ions in aqueous solution, **characterised in that** the ions can diffuse through the measuring electrode (5), whereas the measuring electrode (5) is impermeable to the liquid used as solvent.

2. Measuring electrode (1) as claimed in Claim 1, **characterised in that** the contact means (6) is a liquid, a gel, a foam or a paste.

3. Measuring electrode arrangement (1) as claimed in at least one of the preceding claims, **characterised in that** in order to fix the measuring electrode arrangement (1) on the measurement object (2) an adhesive layer (3.1, 3.2) is disposed on the side of the measuring electrode arrangement (1) facing the measurement object (2).

4. Measuring electrode arrangement (1) as claimed in at least one of the preceding claims, **characterised in that** the storage chamber (7) is delimited by a plastics layer (9).

5. Measuring electrode arrangement (1) as claimed in at least one of the preceding claims, **characterised by** at least one electrical shielding (11) which is made from electrically conductive material and is electrically insulated against the measuring electrode (5).

6. Measuring electrode (1) as claimed in Claim 5, **characterised by** a plurality of measuring electrodes (5) which are electrically insulated from one another.

7. Measuring electrode (1) as claimed in Claim 6, **characterised by** a plurality of shieldings (11) which are electrically insulated from one another, wherein the shieldings (11) are each disposed on one of the measuring electrodes (5).

8. Measuring electrode arrangement (1) as claimed in Claim 6, **characterised by** a common electrical shielding (11) for the measuring electrodes (5).

9. Measuring electrode arrangement (1) as claimed in at least one of Claims 5 to 8, **characterised in that** the shielding (11) is disposed on the side of the measuring electrode (5) facing away from the measurement object (2).

10. Measuring electrode arrangement (1) as claimed in at least one of Claims 5 to 9, **characterised in that** the measuring electrodes (5) are mounted on a belt-like electrode support (8), wherein the electrode support (8) can be expanded in order to set the electrode gap.

11. Use of a measuring electrode (1) as claimed in at least one of the preceding claims for electric impedance tomography.

## Revendications

1. Dispositif d'électrodes de mesure (1) pour la tomographie à électro-impédance, comprenant au moins une électrode de mesure (5) pour l'établissement d'un contact électrique avec un objet à mesurer (2), un espace de réserve (7) étant disposé sur le côté, opposé à l'objet à mesurer (2) de l'électrode de mesure (5), lequel espace contient un moyen de contact (6) pour la réduction de la résistance de passage électrique entre l'électrode de mesure (5) et l'objet à mesurer (2), l'électrode de mesure (5) étant au moins partiellement perméable pour le moyen de contact (6), alors que le moyen de contact contient des ions dans une solution aqueuse,
**caractérisé en ce que**,
les ions peuvent diffuser à travers l'électrode de mesure (5), alors que l'électrode de mesure (5) est imperméable pour le liquide utilisé comme solvant.

2. Dispositif d'électrodes de mesure (1) selon la revendication 1, **caractérisé en ce que** le moyen contact (6) est un liquide, un gel, une mousse ou une pâte.

3. Dispositif d'électrodes de mesure (1) selon au moins l'une quelconque des revendications précédentes, **caractérisé en ce que** une couche collante (3.1, 3.2) est disposée sur le côté, tourné vers l'objet à mesurer (2), du dispositif d'électrodes de mesure (1), afin de fixer le dispositif d'électrodes de mesure (1) sur l'objet à mesurer (2).

4. Dispositif d'électrodes de mesure (1) selon au moins l'une quelconque des revendications précédentes, **caractérisé en ce que** l'espace de réserve (7) est limité par une couche en matière plastique (9).

5. Dispositif d'électrodes de mesure (1) selon au moins l'une quelconque des revendications précédentes, **caractérisé par** au moins un blindage (11) électrique, qui est à base d'un matériau électroconducteur et est isolé électriquement par rapport à l'électrode de mesure (5).

6. Dispositif d'électrodes de mesure (1) selon la revendication 5, **caractérisé par** plusieurs électrodes de mesure (5) isolées électriquement les unes par rapport aux autres.

7. Dispositif d'électrodes de mesure (1) selon la revendication 6, **caractérisé par** plusieurs blindages (11) isolés électriquement les uns par rapport aux autres, les blindages (11) étant disposés chacun sur l'une des électrodes de mesure (5).

8. Dispositif d'électrodes de mesure (1) selon la revendication 6, **caractérisé par** un blindage (11) électrique commun pour les électrodes de mesure (5).

9. Dispositif d'électrodes de mesure (1) selon au moins l'une quelconque des revendications 5 à 8, **caractérisé en ce que** le blindage (11) est disposé sur le côté, opposé à l'objet à mesurer (2), de l'électrode de mesure (5).

10. Dispositif d'électrodes de mesure (1) selon au moins l'une quelconque des revendications 5 à 9, **caractérisé en ce que** les électrodes de mesure (5) sont placées sur un porte-électrode (8) en forme de ceinture, le porte-électrode (8) étant extensible pour le réglage de l'espacement des électrodes.

11. Utilisation d'un dispositif d'électrodes de mesure (1) selon au moins l'une quelconque des revendications précédentes, pour la tomographie à électro-impédance.
